Europäisches Patentamt

European Patent Office

Office européen des brevets

(19)

(11) Numéro de publication : **0 041 441**
**B1**

(12) **FASCICULE DE BREVET EUROPÉEN**

(45) Date de publication du fascicule du brevet :
**20.04.83**

(21) Numéro de dépôt : **81400835.5**

(22) Date de dépôt : **26.05.81**

(51) Int. Cl.³ : **C 07 C 37/60**, C 07 C 41/26,
C 07 C 43/23, C 07 C 39/08,
C 07 B 29/00

(54) **Procédé de méta-hydroxylation des alkyl-phénols et de leurs éthers en milieu superacide.**

(30) Priorité : 03.06.80 FR 8012261

(43) Date de publication de la demande :
**09.12.81 Bulletin 81/49**

(45) Mention de la délivrance du brevet :
**20.04.83 Bulletin 83/16**

(84) Etats contractants désignés :
**BE DE FR GB IT NL**

(56) Documents cités :
**FR A 2 216 254**
**FR A 2 263 215**
**FR A 2 263 217**
**US A 3 600 447**
**US A 3 816 545**

Le dossier contient des informations techniques présentées postérieurement au dépôt de la demande et
ne figurant pas dans le présent fascicule.

(73) Titulaire : **P C U K PRODUITS CHIMIQUES UGINE
KUHLMANN**
**Service Propriété Industrielle Tour Manhattan**
**F-92087 Paris La Défense 2 Cedex 21 (FR)**

(72) Inventeur : **Jouannetaud, Marie-Paule**
**8, Allée du Nivernais**
**F-86000 Poitiers (FR)**
Inventeur : **Gesson, Jean-Pierre**
**8, rue Alexandre Dumas**
**F-86000 Poitiers (FR)**
Inventeur : **Jacquesy, Jean-Claude**
**46, rue du Planty Bruxerolles**
**F-86000 Poitiers (FR)**

# 0 041 441

Procédé de méta-hydroxylation des alkyl-phénols et de leurs éthers en milieu superacide

La présente invention concerne un procédé d'hydroxylation par le peroxyde d'hydrogène des alkyl-phénols et de leurs éthers, en milieu superacide. Ce procédé permet d'introduire sur le noyau aromatique une nouvelle fonction phénol en position méta- par rapport à la précédente ou à la fonction éther de phénol.

Les procédés connus d'hydroxylation radicalaire directe du phénol par le réactif de FENTON (peroxyde d'hydrogène + ions ferreux) conduisent essentiellement à des mélanges de pyrocatéchine et d'hydroquinone, c'est-à-dire que l'hydroxylation se fait préférentiellement en ortho- et en para- de la fonction phénol. Il ne se forme que de très faibles quantités de résorcine (hydroxylation en méta-). De même la photolyse de solutions aqueuses de peroxyde d'hydrogène en présence de para-crésol à pH = 3-8 fournit principalement de la méthyl-4-pyrocatéchine (dihydroxy-3,4-méthyl-1-benzène). De tels procédés sont décrits par exemple dans l'ouvrage « Methoden der Organischen Chemie » (HOUBEN-WEYL), Vierte Auflage, 6/1c, pages 30 à 34.

On a également étudié l'hydroxylation ionique des phénols au moyen de peroxyde d'hydrogène et d'acides carboxyliques, en présence d'un acide minéral fort. Il se forme intermédiairement un peracide organique, qui est un oxydant très puissant et qui a tendance à dégrader les diphénols formés. Ce procédé donne également essentiellement les dérivés dihydroxylés en ortho- et en para-, et ne fournit pratiquement pas de dérivé méta.

D'autres procédés d'hydroxylation ionique du phénol utilisent comme catalyseur des acides minéraux forts, conduisant à la formation d'ions peroxonium. Ces procédés permettent de modifier dans une certaine mesure le rapport pyrocatéchine/hydroquinone, mais ne conduisent pas non plus à la formation de résorcine (voir par exemple l'article : « Hydroquinone et pyrocatéchine par hydroxylation directe du phénol », Chimie-Actualités, 3 novembre 1976, pages 47 à 49).

Dans l'acide fluorhydrique anhydre comme solvant, le phénol est également hydroxylé par le peroxyde d'hydrogène en un mélange d'hydroquinone et de pyrocatéchine (J. Org. Chem., 35, 4028, 1970).

De même, l'hydroxylation ionique du para-crésol donne 20 % de méthyl-hydroquinone et 41 % de méthyl-4-pyrocatéchine (HOUBEN-WEYL, loc. cit. p. 34).

Des essais d'hydroxylation d'hydrocarbures aromatiques par le peroxyde d'hydrogène en milieu superacide ont été rapportés par G. A. OLAH et ses collaborateurs (« Oxyfunctionalization of hydrocarbons-8-Electrophilic hydroxylation of benzene, alkylbenzenes and halobenzenes with $H_2O_2$ in superacids », J. Org. Chem., 43, 865, 1978). Le toluène conduit ainsi, avec un rendement de 67 %, à un mélange de crésols, qui présentent un rapport o/m/p = 71/6/23.

C'est donc de manière tout-à-fait inattendue, au vu de l'art antérieur, que la demanderesse a découvert que les alkylphénols et leurs éthers peuvent être hydroxylés préférentiellement, en méta- de la fonction phénol ou éther, par le peroxyde d'hydrogène dans un milieu superacide liquide, à des températures allant de 0 à − 80 °C.

La présence d'un ou plusieurs substituants alkyls sur le noyau aromatique du phénol ou de l'éther de phénol est nécessaire à la bonne marche de la réaction. Le phénol ordinaire, dans les mêmes conditions d'hydroxylation, ne conduit qu'à un mélange de diphénols, constitué principalement d'hydroquinone et de pyrocatéchine ; il ne se forme que très peu de résorcine. De même, l'anisole conduit à un mélange de para-hydroxy-anisole et d'hydroquinone.

Les substituants alkyls portés par le noyau aromatique du phénol ou de l'éther phénolique peuvent être des radicaux méthyl, éthyl, n-propyl, isopropyl, n-butyl, isobutyl, etc. ou des radicaux cycloalkyls, contenant de 6 à 10 atomes de carbone.

Les radicaux alkyls liés au noyau aromatique par un carbone tertiaire, comme les radicaux tertio-butyls, sont exclus, car dans les conditions de la réaction, les composés phénoliques substitués par ces radicaux subissent une décomposition, avec dans certains cas un déplacement du substituant tertio-alkylé. Ainsi le para-tertio-butyl-phénol conduit à un mélange de phénol et d'hydroquinone, tandis que le ditertio-butyl-2,6-phénol donne un mélange de phénol, d'hydroquinone et de p-tertio-butyl-phénol.

Le milieu superacide liquide dans lequel se déroule la réaction est constitué par des produits comme « l'acide magique » $FSO_3H\text{-}SbF_5$, l'acide fluoroantimonique $HF\text{-}SbF_5$, le complexe acide trifluorométhanesulfonique-pentafluorure d'antimoine $CF_3SO_3H\text{-}SbF_5$, le complexe acide fluorhydrique-trifluorure de bore $HF\text{-}BF_3$, etc., qui ont des acidités estimées sur l'échelle logarithmique de HAMMETT atteignant environ − 25, alors que les valeurs correspondantes d'acidité sont de − 11 pour l'acide sulfurique à 100 % et de − 10 pour l'acide fluorhydrique à 100 %. Ces superacides liquides ont donc des acidités jusqu'à $10^{14}$ fois plus élevées que celles des acides minéraux forts habituels.

Le peroxyde d'hydrogène utilisé dans le procédé selon l'invention est sous forme de solutions aqueuses commerciales, dont le titre en $H_2O_2$ peut aller de 20 à 98 % en poids. La proportion de superacide par rapport au substrat à hydroxyler peut varier dans de larges limites. Elle est en général d'autant plus élevée que la solution de peroxyde d'hydrogène est plus diluée. On travaille en général avec un excès de peroxyde d'hydrogène par rapport à la stoechiométrie.

La réaction d'hydroxylation selon l'invention est rapide et dure le plus souvent de quelques minutes à une demi-heure. Elle conduit en général, avec de bons rendements, au dérivé métahydroxylé désiré, sans réaction secondaire. Cependant dans le cas des éthers phénoliques, où la fonction phénol est substituée par un groupement éthyle ou un groupement supérieur, on observe parfois un clivage de la fonction éther, avec régénération de la fonction phénol. Le radical détaché de la fonction éther peut dans certaines conditions alkyler le noyau aromatique, parfois en s'isomérisant. C'est ainsi que l'éther éthylique du p-crésol donne la méthyl-2-éthyl-3-résorcine et que l'éther n-propylique du p-crésol donne la méthyl-2-isopropyl-3-résorcine.

On peut obtenir aussi parfois de faibles quantités de dérivé trihydroxylé en méta.

Le mode opératoire général suivi pour mettre en œuvre le procédé selon l'invention est très simple : dans un récipient en poly(tétrafluoréthylène), refroidi à la température désirée et muni d'une agitation magnétique, on introduit successivement le superacide liquide, la solution aqeuse de peroxyde d'hydrogène et le phénol ou l'éther de phénol à hydroxyler. Le mélange homogénéisé est maintenu pendant le temps désiré à la température choisie, puis versé sur un mélange d'eau, de glace et de carbonate acide de sodium. Après neutralisation, la solution est extraite trois fois à l'éther. La phase éthérée est lavée à l'eau jusqu'à neutralité, puis séchée sur du sulfate de sodium anhydre. Après évaporation de l'éther, le produit brut de la réaction est filtré sur un gel de silice et analysé par chromatographie en phase vapeur.

Lorsque l'on utilise comme superacide le complexe $HF-SbF_5$, on le dilue en général d'un volume équivalent d'acide fluorhydrique anhydre. On utilise par exemple $1,7$ cm$^3$ d'acide fluorhydrique et $1,7$ cm$^3$ de complexe $HF-SbF_5$ par millimole de phénol ou d'éther de phénol. Si l'hydroxylation est réalisée avec du peroxyde d'hydrogène à 95 % en poids, on utilise $1,4$ équivalent de $H_2O_2$ pour les phénols (temps de réaction de 30 minutes à $-41$ °C) et $1,2$ équivalent de $H_2O_2$ pour les éthers de phénol (temps de réaction de 5 minutes à $-41$ °C). Si l'on emploie pour l'hydroxylation une solution aqueuse de peroxyde d'hydrogène à 30 % en poids, on utilise les mêmes équivalents de $H_2O_2$ que précédemment, mais le rapport molaire $SbF_5$/substrat est porté à 16-17. Les temps de réaction sont aussi de 30 minutes à $-41$ °C pour les phénols et de 5 minutes à $-41$ °C pour les éthers de phénols.

Le complexe $HF-BF_3$ est formé en faisant barboter à $-41$ °C dans de l'acide fluorhydrique anhydre un courant de trifluorure de bore, jusqu'à saturation, pendant environ 5 minutes.

Les complexes $CF_3SO_3H-SbF_5$ et le complexe $FSO_3H-SbF_5$ sont formés en mélangeant à $-41$ °C les quantités voulues d'acide sulfonique et de pentafluorure d'antimoine.

Dans les tableaux suivants sont rassemblés divers exemples, qui illustrent l'invention sans la limiter. Le tableau I concerne les exemples portant sur divers alkylphénols. Le tableau II concerne les exemples portant sur divers éthers d'alkylphénols.

### Tableau I

| N° de l'exemple | Produit de départ | Milieu superacide | Température °C | Taux de transformation % | Produits de la réaction |
|---|---|---|---|---|---|
| 1 | p-crésol | $HF-SbF_5$ | $-41$ | 78 | méthyl-4-résorcine |
| 2 | diméthyl-2,4-phénol | $HF-SbF_5$ | $-41$ | 90 | diméthyl-4,6-résorcine (58 %) diméthyl-2,4-résorcine (32 %) |
| 3 | diméthyl-3,4-phénol | $HF-SbF_5$ | $-41$ | 88 | diméthyl-4,5-résorcine |
| 4 | diméthyl-2,6-phénol | $HF-SbF_5$ | $-41$ | $<30$ | diméthyl-2,4-résorcine (quelques %) diméthyl-2,4-phloroglucinol (4 %) |
| 5 | ortho-crésol | $HF-SbF_5$ | $-40$ | 28 | méthyl-hydroquinone (16,8 %) méthyl-4-résorcine (3,9 %) méthyl-3-pyrocatéchine (4,2 %) méthyl-2-résorcine (3,1 %) |
| 6 | méthyl-4-éthyl 3 phénol | $HF-SbF_5$ | $-40$ | 57 | méthyl-2-éthyl-3-résorcine |

3

Tableau II

| N° de l'exemple | Produit de départ | Milieu superacide | Température °C | Taux de transformation % | Produits de la réaction |
|---|---|---|---|---|---|
| 7 | méthyl-4-anisole | HF-SbF$_5$ | − 41 | 75 | Hydroxy-3-méthyl-4-anisole |
| 8 | méthyl-4-anisole | HF-SbF$_5$ | 0 | 75 | Hydroxy-3-méthyl-4-anisole |
| 9 | méthyl-4-anisole | CF$_3$SO$_3$H-SbF$_5$ | − 41 | 75 | Hydroxy-3-méthyl-4-anisole |
| 10 | méthyl-4-anisole | HF-BF$_3$ | − 41 | 52 | para-crésol (26 %) Hydroxy-2-méthyl-4-anisole (10 %) Hydroxy-3-méthyl-4-anisole (16 %) |
| 11 | diméthyl-2,4-anisole | HF-SbF$_5$ | − 41 | 84 | Hydroxy-5-diméthyl-2,4-anisole |
| 12 | diméthyl-3,4-anisole | HF-SbF$_5$ | − 41 | 75 | Hydroxy-5-diméthyl-3,4-anisole |
| 13 | diméthyl-2,6-anisole | HF-SbF$_5$ | − 41 | 85 | Hydroxy-3-diméthyl-2,6-anisole (55 %) Diméthyl-2,4-résorcine (22 %) Dihydroxy-3,5-diméthyl-2,6-anisole (8 %) |
| 14 | éther éthylique du p-crésol | HF-SbF$_5$ | − 41 | 68 | méthyl-4 éthyl-5-résorcine |
| 15 | éther n-propylique du p-crésol | HF-SbF$_5$ | − 41 | 50 | méthyl-4-isopropyl-5-résorcine |

## Revendications

1. Procédé d'hydroxylation des alkyl-phénols et de leurs éthers, en position méta par rapport à la fonction phénol ou éther, caractérisé en ce que l'on fait réagir sur le composé phénolique une solution aqueuse de peroxyde d'hydrogène en présence d'un superacide liquide, à une température comprise entre 0 et − 80 °C.

2. Procédé selon la revendication 1, caractérisé en ce que la solution aqueuse de peroxyde d'hydrogène a une concentration pondérale comprise entre 20 et 98 %.

3. Procédé selon l'une ou l'autre des revendications 1 et 2, caractérisé en ce que le superacide liquide est le complexe HF-SbF$_5$.

4. Procédé selon l'une ou l'autre des revendications 1 et 2, caractérisé en ce que le superacide liquide est le complexe CF$_3$SO$_3$H-SbF$_5$.

5. Procédé selon l'une ou l'autre des revendications 1 et 2, caractérisé en ce que le superacide liquide est le complexe HF-BF$_3$.

6. Procédé selon l'une quelconque des revendications 1 à 5, caractérisé en ce que le phénol ou l'éther phénolique de départ portent sur le noyau aromatique de 1 à 4 substituants alkyls ou cycloalkyls, non liés au noyau par un carbone tertiaire, contenant de 1 à 10 atomes de carbone et disposés de manière à laisser au moins une position méta libre par rapport à la fonction phénol ou éther.

7. Procédé selon l'une quelconque des revendications 1 à 6, caractérisé en ce que l'éther phénolique de départ porte sur la fonction éther un radical alkyl ou cycloalkyl contenant de 1 à 10 atomes de carbone.

## Claims

1. Process for the hydroxylation of alkylphenols and their ethers, in the meta-position relative to the phenol or ether group, characterised in that an aqueous solution of hydrogen peroxide is reacted with the phenol compound, in the presence of a liquid superacid, at a temperature of between 0 and − 80 °C.

2. Process according to Claim 1, characterised in that the aqueous solution of hydrogen peroxide has a concentration by weight of between 20 and 98 %.

3. Process according to one or other of Claims 1 and 2, characterised in that the liquid superacid is the complex HF-SbF$_5$.

4. Process according to one or other of Claims 1 and 2, characterised in that the liquid superacid is the complex CH$_3$SO$_3$H-SbF$_5$.

5. Process according to one or other of Claims 1 and 2, characterised in that the liquid superacid is the complex HF-BF$_3$.

6. Process according to any one of Claims 1 to 5, characterised in that the starting phenol or phenol ether carries, on the aromatic nucleus, from 1 to 4 alkyl or cycloalkyl substituents not bonded to the nucleus by a tertiary carbon, containing from 1 to 10 carbon atoms and arranged in such a way as to leave free at least one meta position relative to the phenol or ether group.

7. Process according to any one of Claims 1 to 6, characterised in that the starting phenol ether carries, on the ether group, an alkyl or cycloalkyl radical containing from 1 to 10 carbon atoms.

## Ansprüche

1. Verfahren zur Hydroxylierung von Alkylphenolen und ihren Äthern in meta-Stellung zur Phenol- oder Äthergruppe, dadurch gekennzeichnet, daß man auf die phenolische Verbindung bei einer Temperatur von 0 bis − 80 °C eine wässrige Wasserstoffperoxidlösung in Gegenwart einer flüssigen Supersäure einwirken läßt.

2. Verfahren nach Anspruch 1, dadurch gekennzeichnet, daß die wässrige Wasserstoffperoxidlösung eine Konzentration von 20 bis 98 Gewichtsprozent hat.

3. Verfahren nach Anspruch 1 oder 2, dadurch gekennzeichnet, daß die flüssige Supersäure der HF-SbF$_5$-Komplex ist.

4. Verfahren nach Anspruch 1 oder 2, dadurch gekennzeichnet, daß die flüssige Supersäure der CF$_3$SO$_3$H-SbF$_5$-Komplex ist.

5. Verfahren nach Anspruch 1 oder 2, dadurch gekennzeichnet, daß die flüssige Supersäure der HF-BF$_3$-Komplex ist.

6. Verfahren nach irgendeinem der Ansprüche 1 bis 5, dadurch gekennzeichnet, daß das eingesetzte Phenol oder der eingesetzte Phenoläther an dem aromatischen Kern 1 bis 4 Alkyl-oder Cycloalkylgruppen tragen, die mit dem Kern nicht über ein tertiäres Kohlenstoffatom verbunden sind, 1 bis 10 Kohlenstoffatome besitzen und in der Weise angeordnet sind, daß sie wenigstens eine meta-Stellung gegenüber der Phenol- oder Äthergruppe freilassen.

7. Verfahren nach irgendeinem der Ansprüche 1 bis 6, dadurch gekennzeichnet, daß der eingesetzte Phenoläther an der Äthergruppe einen Alkyl- oder Cycloalkylrest mit 1 bis 10 Kohlenstoffatomen trägt.